# EUROPEAN PATENT SPECIFICATION

(11) **EP 1 110 541 B1**
(45) Date of publication and mention of the grant of the patent: **20.10.2004**
(21) Application number: 00311631.6
(22) Date of filing: 22.12.2000
(51) Int. Cl.: A61K 9/19

(54) **Controlled release composition comprising a sustained release layer and a fast release layer**
Zusammensetzung zur kontrollierten Wirkstoffabgabe enthaltend eine verzögert feisetzende Schicht und eine schnell freisetzende Schicht
Composition à libération contrôlée comprenant une couche à libération prolongée at une couche à libération rapide

(30) Priority: 23.12.1999 US 471825
(43) Date of publication of application: 27.06.2001
(73) Proprietor: JOHNSON & JOHNSON CONSUMER COMPANIES, INC., Skillman, NJ 08558 (US)
(72) Inventor: Lin, Shun Y, Plainsboro, NJ 08536 (US); Wearley, Lorraine L., Westfield, NJ 07090 (US); Gole, Dilip J., Plainsboro, NJ 08536 (US); Posage, Gary W., New Hope, Pennsylvania 18938 (US); Wilkinson, Paul K., Doylestown, Pennsylvania 18901 (US)
(74) Representative: Fisher, Adrian John

(56) References cited:
- EP-A- 0 090 997
- EP-A- 0 220 670
- EP-A- 0 747 045
- WO-A-93/02662
- WO-A-99/33448
- US-A- 4 122 157
- US-A- 4 915 953
- US-A- 5 085 865
- US-A- 5 891 458
- US-A- 6 004 582
- CHEMICAL ABSTRACTS, vol. 127, no. 22, 1 December 1997 (1997-12-01) Columbus, Ohio, US; abstract no. 311405, IWATA, MASANORI ET AL: "Sustained-release double- layered progesterone suppository for luteal - support therapy" XP002162841 & YAKUGAKU ZASSHI (1997), 117(9), 629-635 ,

## Description

### FIELD OF THE INVENTION

The present invention relates to a composition which provides fast and sustained delivery of a pharmaceutically active agent. The present invention also relates to methods of preparing the same.

### BACKGROUND OF THE INVENTION

Freeze-drying, also known as lyophilization, is a well-known method of drying heat-sensitive materials in order to increase product stability and shelf life. Products containing active ingredients, such as pharmaceuticals, nutrients, diagnostics, fertilizers, and insecticides are frequently prepared by freeze-drying aqueous solutions or suspensions containing these active ingredients. Generally, freeze-drying involves freezing a material and then sublimating it under high vacuum.

Conventional freeze-drying processes often cause cracking of the freeze-dried preparation and meltback. Cracking typically is caused by stresses during ice crystallization. Meltback occurs when the heat required for drying melts the frozen material, defeating the purpose of the freeze-drying process. Meltback can result in interfacial penetration of layers in multi-layered dosage forms. To avoid both cracking and meltback during freeze-drying, small amounts of material of limited thickness are typically dried at one time or, alternatively, at very low temperatures. Sublimation at very low temperatures, however, usually requires a relatively long period of time.

Freeze-drying methods generally yield products which disintegrate easily and are often sticky and crumbly when handled. Various freeze-drying and packaging methods have been employed in attempts to circumvent this problem. However, tablets produced by such methods usually are still susceptible to sticking and crumbling if transferred to other packaging.

Conventional freeze-drying methods also do not produce products which have uniform porosity. Uniform porosity in a freeze-dried product is important for controlling the release of active agents from the freeze-dried dosage form.

In the area of pharmaceuticals, dosage forms which can provide a fast and sustained treatment are often of critical importance for patient's compliance, especially for patients using over-the-counter medicines. A dosage form with a fast release layer requires less time to reach the effective drug concentration in the target area than a conventional sustained release dosage form. A dosage form with a sustained release layer is designed to maintain a therapeutic drug concentration in the target area for relatively long periods of time. Presently, patients often have to be treated with two or more different dosage forms in order to obtain a fast and sustained therapeutic effect. Therefore, a single dosage form which provides a fast and sustained therapeutic effect would significantly improve patients' compliance.

Vaginal dosage forms such as creams, gels, suppositories, and ovules are well known in the art. These dosage forms, however, tend to spread out readily and often inadvertently discharge from the cavity, making such products inconvenient and messy to use. Thus, there is a need for a dosage form which provides both a fast and sustained therapeutic effect in target areas, more particularly, in areas such as vaginal cavity, where multi-dosing is inconvenient and messy. Lyophilized suppository compositions are disclosed in US-A-5 891 458 and EP-A-0 747 045.

### SUMMARY OF THE INVENTION

The present invention provides a composition comprising a sustained release layer and a fast release layer. The sustained release layer comprises a water-soluble polymer and a first pharmaceutically active agent. The fast release layer comprises a matrix forming agent and a second pharmaceutically active agent. Generally, the composition provides fast and sustained (or controlled) release of a pharmaceutically active agent for at least 6 hours and preferably for at least 1 to 3 days. The composition may be incorporated into a dosage unit form, such as a vaginal insert.

The aforementioned composition is obtainable by (a) preparing a first aqueous solution containing a water-soluble polymer and a first pharmaceutically active agent; (b) preparing a second aqueous solution containing a matrix forming agent and a second pharmaceutically active agent; (c) pouring the first and second aqueous solutions into a container; and (d) freeze-drying the solution in the container to produce the composition.

### DETAILED DESCRIPTION OF THE INVENTION

The composition of the present invention comprises a sustained release layer and a fast release layer. Generally, the composition provides fast and sustained (or controlled) release of a pharmaceutically active agent for at least 6 hours and preferably for at least 1 to 3 days. The composition is particularly useful as a vaginal insert for treatment of vaginal diseases without multi-dosing.

The sustained release layer comprises a water-soluble polymer and a first pharmaceutically active agent. Suitable water soluble polymers include, but are not limited to, celluloses, cellulose ethers, or derivatives thereof, such as those disclosed in U.S. Patent No. 4,615,697 and polycarbophils; polycarboxylated vinyl polymers, such as polyacrylic acid polymers optionally crosslinked with polyalkenyl polyethers, such as, for example, Carbopol 434, Carbopol 934P, Carbopol 940, and Carbopol 941, all of which are available from B. F. Goodrich of Cincinnati, OH; polyurethanes; gelatins; polysaccharide gums, such as natural plant exudates, including, but not limited to, karaya gum and ghatti gum; seed gum, such as guar gum, locust bean gum, and psyllium seed gum; crosslinked alginate gum gel, such as those disclosed in U.S. Patent No. 3,640,741; and any combination of any of the foregoing. Preferred water-soluble polymers include, but are not limited to, polyurethanes, gelatins, hydroxypropylmethylcellulose, sodium carboxymethylcellulose, methylcellulose, hydroxyethylcellulose, hydroxypropylcellulose, hydroxyethylmethylcellulose, hydroxyethylethylcellulose, hydroxypropylethylcellulose, carbopol, polyvinyl alcohol and derivatives thereof, dextran, chitosan and derivatives thereof, starches and derivatives thereof, polyacrylamides, polyacrylates, agar, collagen, fibronectin, alginic acid, pectin, hyaluronic acid, and any combination of any of the foregoing. The matrix forming agent forms a matrix and aids in dispersing the pharmaceutically active agent in the layer.

The sustained release layer preferably includes a fatty acid or a mixture of fatty acids, such as a hydrogenated vegetable oil. The fatty acid accelerates disintegration of the layer and release of the pharmaceutically active agent. Preferred fatty acids melt at about body temperature. Two preferred hydrogenated vegetable oils are Wecobee FS™ and Wecobee M™ available from Stepan Company of Northfield, IL.

Suitable pharmaceutically active agents include, but are not limited to, anti-fungal agents, anti-bacterial agents, nutrients, vitamins, minerals, diagnostics, fertilizers, insecticides, and any combination of any of the foregoing. Other suitable pharmaceutically active agents include, but are not limited to, those which have a prophylactic purpose, such as prevention of pregnancy and sexually transmitted diseases. Preferred pharmaceutically active agents include, but are not limited to, metronidazole; terconazole; miconazole nitrate; chlorpheniramine maleate; pseudophedrine; detromethorphan; meclizine dihydrochloride; haloperidol; albuterol sulfate; dimenhydrinate; benzodiazepines, such as diazepam, lorazepam, and congeners thereof; and any combination of any of the foregoing. A more preferred pharmaceutically active agent is metronidazole. The pharmaceutically active agent may be coated with any coating agent known in the art. Preferably the pharmaceutically active agent is coated with a coating agent which protects it from solvents and other chemicals and environmental conditions which could dissolve or deteriorate the pharmaceutically active agent before reaching its intended target. The coating agent may also mask the flavor and/or odor of the pharmaceutically active agent. Suitable coating agents include, but are not limited to, fatty acids; glycerides, including, but not limited to, triglycerides; and any combination of any of the foregoing.

The sustained release layer generally comprises from about 5 to about 70% by weight, preferably from about 10 to about 50% by weight, and more preferably from about 10 to about 30% by weight of water-soluble polymer, based upon 100% total weight of sustained release layer.

The sustained release layer preferably comprises up to about 15% by weight and more preferably from about 5 to about 10% by weight of fatty acid, based upon 100% total weight of sustained release layer.

The weight ratio of fatty acid to water-soluble polymer preferably ranges from about 1:10 to about 3:5 and is more preferably about 2:5.

The sustained release layer generally comprises a therapeutic effective amount of pharmaceutically active agent. The sustained release layer preferably contains from about 15 to about 95% by weight and more preferably from about 50 to about 85% by weight of pharmaceutically active agent, based upon 100% total weight of sustained release layer.

The sustained release layer may include other adjuvants, such as preservatives, flavorants, antioxidants, surfactants, sweeteners, viscosity enhancers, colorants, fragrances, plasticizers, lubricants, fillers, binders, wetting agents, penetration agents, pH adjusters, disintegrants, excipients, or any combination of any of the foregoing. Since the water uptake of the sustained release layer is typically not significant, preservatives are generally not required to enhance the stability of the composition.

The sustained release layer is typically flexible and muco-adherent.

Generally, the sustained release layer provides a sustained release of a therapeutically effective amount of the first pharmaceutically active agent. Preferably, the sustained release layer provides a sustained release of the first pharmaceutically active agent for at least 6 hours and more preferably for at least about 1 to 3 days.

The fast release layer comprises a matrix forming agent and a second pharmaceutically active agent. Suitable matrix forming agents include, but are not limited to, animal and vegetable protein derivatives, such as gelatins, dextrins, soy, and wheat end psyllium seed proteins; gums, such as acacia, guar, agar, and xantham; polysaccharides; alginates; carboxymethylcelluloses; carrageenans; dextrans; pectins; polyvinylpyrrolidones; polyacrylic acids; polypeptide/protein complexes, such as gelatin-acacia complexes; polypeptide/polysaccharide complexes; sugars, such as mannitol, dextrose, lactose, galactose, and cyclodextrin; inorganic salts, such as sodium phosphate, sodium chloride, and aluminum silicates; and amino acids having from about 2 to about 12 carbon atoms, such as glycine, L-alanine, L-aspartic acid, L-glutamic acid, L-hydroxyproline, L-isoleucine, and L-leucine, L-phenylalanine; and any combination of any of the foregoing. A preferred amino acid is glycine.

Preferably, the fast release layer comprises a gelatin, pectin, soy fiber protein, or a mixture thereof and an amino acid having from about 2 to about 12 carbon atoms. More preferably, the fast release layer comprises a gelatin, pectin, or a mixture thereof and an amino acid having from about 2 to about 12 carbon atoms.

The pharmaceutically active agent may be any of the aforementioned pharmaceutically active agents. The pharmaceutically active agent in the fast release layer may be the same or different than the pharmaceutically active agent in the sustained release layer.

The fast release layer generally comprises from about 0.5 to about 15% by weight, preferably from about 0.5 to about 10% by weight, and more preferably from about 4 to about 10% by weight of matrix forming agents, based upon 100% total weight of fast release layer. According to one preferred embodiment, the fast release layer comprises (A) from about 4 to about 8% by weight of a matrix forming agent and (B) from about 1 to about 20% by weight of an amino acid having from about 2 to about 12 carbon atoms, based upon 100% total weight of fast release layer.

The fast release layer generally comprises a therapeutic effective amount of pharmaceutically active agent. The fast release layer preferably contains from about 15 to about 95% by weight and more preferably from about 60 to about 90% by weight of pharmaceutically active agent, based upon 100% total weight of fast release layer.

According to a preferred embodiment, the fast release layer contains from about 4 to about 8% by weight of a matrix forming agent, from about 3 to about 5% by weight of at least one amino acid, and from about 2 to about 5% by weight of mannitol, based upon 100% total weight of fast release layer.

The fast release layer may include any of the aforementioned adjuvants.

Generally, the fast release layer provides fast release of the second pharmaceutically active agent. Preferably, the fast release layer releases a therapeutically effective amount of the second pharmaceutically active agent within about 4 minutes and more preferably within about 2 minutes.

The fast release layer may surround the sustained release layer. Also, both the fast release layer and the sustained release layer may be on the surface of the composition.

The density of the composition typically ranges from about 0.1 to about 0.5 g/cc. The dissolution rate of the composition generally ranges from about 1 to about 30 weight percent per hour, based upon 100% weight of composition.

Generally, the amount of pharmaceutically active agent in the composition is an amount effective to accomplish the purpose for which it is being used. The amount of pharmaceutically active agent is typically a pharmacologically or biologically effective amount. However, the amount can be less than a pharmaceutically or biologically effective amount when the composition is used in a dosage unit form because the dosage unit form may contain a multiplicity of compositions of the present invention or may contain a divided pharmacologically or biologically effective amount. The total effective amount can then be determined in cumulative units containing, in total, a pharmacologically or biologically effective amount of the pharmaceutically active agent. The total amount of pharmaceutically active agent can be determined by those skilled in the art.

The composition may be incorporated into a dosage unit form, such as a vaginal insert. The vaginal insert may be administered digitally or with an applicator inside the vagina, preferably proximate to the cervix. The vaginal insert is typically completely and naturally soluble in the vagina. Since the vaginal insert is typically flexible and muco-adherent, the insert is comfortable to wear and generally does not prematurely discharge from the vagina. Furthermore, the muco-adherent properties of the composition enhance the drug-vaginal contact area and intravaginal retention. This results in increased delivery of the pharmaceutically active agent. The dosage unit form may be a non-aqueous cream, non-aqueous gel, suppository, or ovule.

The composition of the present invention may be prepared as follows. A water-soluble polymer and a first pharmaceutically active agent are added to water and mixed to form a first aqueous solution. The solution may also contain other water-miscible solvents. The solution generally contains from about 1 to about 20% by weight, preferably from about 2 to about 16% by weight, and more preferably from about 2 to about 7% by weight of a water-soluble polymer, based upon 100% weight of total solution. The solution also generally contains from about 1 to about 35% by weight and preferably from about 5 to about 25% by weight of a pharmaceutically active agent, based upon 100% weight of total solution. At concentrations below about 1% by weight of water-soluble polymer, the viscosity of the solution is typically very low, resulting in poor muco-adherent properties and uncontrolled release of the pharmaceutically active agent. A fatty acid and other adjuvants as described above may be added to the first aqueous solution. The solution preferably contains up to about 5% by weight and more preferably from about 1 to about 3% by weight of fatty acid, based upon 100% weight of total solution. Preferably, the solution is mixed to form a homogeneous mixture. The pH of the mixture may be adjusted with a pH adjuster to optimize solubility and stability of the final composition. The solution preferably has a specific gravity ranging from about 1.0 to about 1.2 g/mL.

In a separate container, a matrix forming agent and a second pharmaceutically active agent are added to water and mixed to form a second aqueous solution. The solution may also contain other water-miscible solvents. The matrix forming agent typically aids in dispersing the pharmaceutically active agent, especially when the pharmaceutically active agent is not water soluble. The second solution generally contains from about 0.1 to about 5% by weight, preferably from about 0.1 to about 3% by weight, and more preferably from about 1.5 to about 3% by weight of a matrix forming agent, based upon 100% weight of total solution. The second solution preferably contains from about 1 to about 35% by weight and more preferably from about 5 to about 25% by weight of a pharmaceutically active agent, based upon 100% weight of total solution.

According to one preferred embodiment, the second solution contains at least about 0.1% by weight of matrix forming agent, and an amino acid having from about 2 to about 12 carbon atoms, based upon 100% weight of total second solution. Preferably, the matrix forming agent is gelatin, pectin, soy fiber protein, or a mixture thereof. The amino acid is preferably glycine.

According to another preferred embodiment, the second solution contains from about 1.5 to about 2.5% by weight of a matrix forming agent and from about 0.5 to about 10% by weight of an amino acid having from about 2 to about 12 carbon atoms, based upon 100% weight of total second solution.

According to yet another preferred embodiment, the second solution contains from about 0.1 to about 3% by weight of matrix forming agent, from about 0.5 to about 10% by weight of one or more amino acids, and from about 0.5 to about 10% by weight of mannitol, based upon 100% weight of total second solution. More preferably, the second solution contains from about 1.5 to about 2.5% by weight of matrix forming agent, from about 1.4 to about 1.7% by weight of one or more amino acids, and from about 1.0 to about 1.5% by weight of mannitol, based upon 100% weight of total second solution.

The first and second aqueous solutions are poured into a container, such as a pre-shaped plastic cavity. According to one preferred embodiment, the first aqueous solution is poured into the container before the second aqueous solution is poured. According to another preferred embodiment, the weight ratio of the first solution to the second solution is about 1:1.

The solution in the container is then freeze-dried to form the composition. The solution may be freeze-dried by any method of freeze-drying known in the art. A preferred method of freeze-drying the solution comprises freezing the solution and lyophilizing the frozen solution. Freezing is preferably performed rapidly, such as with a cold gas freezing tunnel. The water in the frozen solution is then removed by lyophilization.

The following examples 13-15, 19, 20 are intended to describe the present invention without limitation. Examples 1-12, 17 and 18 relating to mono-layered compositions do not represent the invention according to the claims.

### Examples 1-3

Fast release layers were prepared from each formulation shown in Table 1 as follows. Gelatin, mannitol, metronidazole, neutralized 0.5% carbopol gel, glycine, and xanthan gum were dissolved in water under constant stirring. The resulting solution was carefully transferred into a 1 mL size mold. The solution was frozen rapidly in a cold gas freezing tunnel. The water in the ice state was then removed by lyophilization to produce the fast release layer.

**Table 1**

| Component | Example 1 (g) | Example 2 (g) | Example 3 (g) |
|---|---|---|---|
| Gelatin | 1.4 | 1.4 | 1.4 |
| Mannitol | 0.9 | 0.9 | 0.9 |
| Metronidazole | 20 | 20 | 20 |
| Neutralized 0.5% Carbopol Gel¹ | 8.0 | 12.0 | 8.0 |
| Glycine | 1.0 | 1.0 | 1.0 |
| Xanthan Gum | 0.075 | - | - |
| Water | 68.6 | 64.7 | 68.7 |

| | | | |
|---|---|---|---|
| ¹ - The neutralized 0.5% carbopol gel is prepared by neutralizing carbopol with 5% w/w sodium hydoxide solution. Carbopol is available in powder form from B.F. Goodrich of Cleveland, OH. | | | |

### Examples 4-12

Sustained release layers were prepared from each formulation shown in Tables 2 and 3 below as follows. Hydroxypropyl-methylcellulose, metronidazole, Wecobee FS™, and Wecobee M™ were dissolved or dispersed in water heated to 50-70° C, under constant stirring. In some cases, the pH of solution was adjusted with lactic acid. The resulting solution was cooled to room temperature and carefully transferred into molds of various sizes. The pH, viscosity, and specific gravity (at room temperature) of the solution was determined and are shown in Tables 2 and 3. The solutions in the molds were frozen rapidly in a cold gas freezing tunnel. The water in the ice state was removed by lyophilization.

**Table 2**

| Component | Example (weight in grams) | | | | |
|---|---|---|---|---|---|
| | 4 | 5 | 6 | 7 | 8⁵ |
| Hydroxypropylmethyl cellulose² | 5 | 5 | 5 | 5 | 7 |
| Metronidazole | 12.5 | 12.5 | 12.5 | 12.5 | 12.5 |
| Lactic Acid (pH adjuster) | - | 0.1 | - | 0.1 | - |
| Wecobee FS™ ³ | - | - | 1.4 | 1.4 | - |
| Wecobee M™ ⁴ | - | - | 0.6 | 0.6 | - |
| Water | 82.5 | 82.4 | 80.5 | 82.4 | 80.5 |
| Viscosity (cPs) | 2000 | 2800 | 2500 | 2600 | 11000 |
| Specific Gravity (g/mL) | 1.07 | 1.04 | 1.04 | 1.04 | 1.07 |
| pH | - | 3.72 | - | 3.74 | - |
| Percentage of Metronidazole in Sustained Release Layer | 71% | 71% | 64% | 64% | 64% |

| | | | | | |
|---|---|---|---|---|---|
| ² - The hydroxypropylmethyl cellulose was HPMC E50LV™ available from Dow Chemical Co. of Midland, MI. | | | | | |
| ³ - Wecobee FS™ is available from Stepan Company of Northfield, IL. | | | | | |
| ⁴ - Wecobee M™ is available from Stepan Company of Northfield, IL. | | | | | |
| ⁵ - The solution in Example 8 was not lyophilized since its viscosity was so high. | | | | | |

**Table 3**

| Component | Example (weight in grams) | | | |
|---|---|---|---|---|
| | 9 | 10 | 11 | 12 |
| Hydroxypropylmethyl cellulose⁶ | 5 | 5 | 5 | 5 |
| Metronidazole | 25.0 | 25.0 | 25.0 | 25.0 |
| Lactic Acid (pH adjuster) | - | 0.1 | - | 0.1 |
| Wecobee FS™ ⁷ | - | - | 1.4 | 1.4 |
| Wecobee M™ ⁸ | - | - | 0.6 | 0.6 |
| Water | 70.0 | 69.9 | 68.0 | 67.9 |
| Viscosity (cPs) | 8300 | 6500 | 7400 | 8400 |
| Specific Gravity (g/mL) | 1.10 | 1.09 | 1.08 | 1.08 |
| pH | - | 3.73 | - | 3.70 |
| Percentage of Metronidazole in Sustained Release Layer | 83% | 83% | 78% | 78% |

| | | | | |
|---|---|---|---|---|
| ⁶ - The hydroxypropylmethyl cellulose was HPMC E50LV™ available from Dow Chemical Co. of Midland, MI. | | | | |
| ⁷ - Wecobee FS™ is available from Stepan Company of Northfield, IL. | | | | |
| ⁸ - Wecobee M™ is available from Stepan Company of Northfield, IL. | | | | |

### Example 13

A multi-layer composition was prepared as follows. The solution of Example 9 (before freeze-drying) was dispensed, cooled to room temperature, and carefully transferred into a 1.0 mL mold. The solution of Example 1 (before freeze-drying) was then dispensed, cooled to room temperature, and carefully transferred into the mold. The weight ratio of the solution of Example 9 to the solution of Example 1 was about 1:1. The mold and its contents were frozen rapidly in a cold gas freezing tunnel. The water in the ice state was removed by lyophilization to produce the multi-layer composition.

### Example 14

A multi-layer composition was prepared as described in Example 13, except the solution of Example 2 was substituted for the solution of Example 1.

### Example 15

A multi-layer composition was prepared as described in Example 13, except the solution of Example 3 was substituted for the solution of Example 1.

### Example 16

The fast release layers, sustained release layers, and the multi-layer compositions prepared in Examples 1-7 and 9-15 were tested as follows.

The dissolution rate was determined for each release layer and composition by a modified USP type I dissolution method (United States Phamacopeia <711>). A dialysis membrane with known molecular weight cut-off and diameter was used instead of a mesh basket for holding the test samples. The membrane limited the amount of dissolution medium which contacted the release layer or composition. This modified dissolution procedure was designed to mimic a vaginal environment where only limited amounts of a medium are typically in contact with the composition. Each release layer and composition was tested in an aqueous medium and in a buffered aqueous medium, which was maintained at a pH of about 4. The normal vaginal pH range is from about 3 to about 5. Metrogel™ available from 3M Pharmaceuticals of Northridge, CA, was also tested as described above. The results are shown in Table 4 below.

The moisture absorption of each release layer and composition was determined by measuring the percentage weight gain after storing the release layer or composition for one month at 25° C and a relative humidity of 60% and at 40° C and a relative humidity of 75%. The results are shown in Table 5 below.

**Table 4**

| Example | Sample Size (mL) | Disintegration (75% disintegrated) | Dissolution in water (% by weight/hour) | Dissolution in buffer (% by weight /hour) |
|---|---|---|---|---|
| 1 | 0.5 | <1 seconds | - | 29% |
| 2 | 0.5 | <15 seconds | - | - |
| 3 | 0.5 | <15 seconds | - | - |
| 4 | 1.0 | <20 minutes | - | 14% |
| 5 | 1.0 | <15 minutes | - | 13% |
| 6 | 1.0 | <2 minutes | - | 13% |
| 7 | 1.0 | <2 minutes | - | 15% |
| 9 | 0.5 | <11 minutes | 14% | 15% |
| 10 | 0.5 | <13 minutes | 13% | 11% |
| 11 | 0.5 | <2 minutes | - | 10% |
| 12 | 1.0 | <2 minutes | 12% | 10% |
| 13 | 1.0 | <10 minutes ⁹ | 12% | 18% |
| 14 | 1.0 | <10 minutes ⁹ | - | 13% |
| 15 | 1.0 | <10 minutes ⁹ | - | 10% |
| Metrogel™ (vaginal) | - | - | 49% | 55% |

| | | | | |
|---|---|---|---|---|
| ⁹ - The fast release layer of the compositions prepared in Examples 13-15 separated from the sustained release layer and disintegrated within about 10-15 seconds. | | | | |

**Table 5**

| Example | Sample Size (mL) | Moisture absorption at 25° C and 60% relative humidity (% w/w gain) | Moisture absorption at 40° C and 75% relative humidity (% w/w gain) |
|---|---|---|---|
| 1 | 0.5 | - | - |
| 2 | 0.5 | - | - |
| 3 | 0.5 | - | - |
| 4 | 1.0 | <0.1% | <2% |
| 5 | 1.0 | <0.1% | <5% |
| 6 | 1.0 | <0.1% | <2% |
| 7 | 1.0 | <0.1% | <2% |
| 9 | 0.5 | <0.1% | <2% |
| 10 | 0.5 | <0.1% | <1% |
| 11 | 0.5 | <0.1% | <4% |
| 12 | 1.0 | <0.1% | <1% |
| 13 | 1.0 | <0.1% | <1% |
| 14 | 1.0 | <0.1% | <1% |
| 15 | 1.0 | <0.1% | <1% |
| Metrogel™ (vaginal) | - | - | - |

### Example 17

A fast release layer was prepared from the formulation shown in Table 6 below as follows. Gelatin, mannitol, terconazole, carbopol, sodium hydroxide, glycine, and simethicone were dissolved in water under constant stirring. The resulting solution was carefully transferred into a 0.5 mL size mold. The solution was frozen rapidly in a cold gas freezing tunnel. The water in the ice state was then removed by lyophilization to produce the fast release layer.

**Table 6**

| Component | % w/w |
|---|---|
| Gelatin | 1.398 |
| Mannitol | 0.900 |
| Terconazole | 20.000 |
| Carbopol | 0.025 |
| Sodium Hydroxide | 0.013 |
| Glycine | 1.000 |
| Simethicone | 0.004 |
| Water | 76.600 |

### Example 18

A sustained release layer was prepared from the formulation shown in Table 7 below as follows. Hydroxypropylmethyl cellulose and terconazole were dissolved or dispersed in water heated to 50-70° C, under constant stirring. The resulting solution was cooled to room temperature and carefully transferred into molds of various sizes. The solutions in the molds were frozen rapidly in a cold gas freezing tunnel. The water in the ice state was removed by lyophilization.

**Table 7**

| Component | % w/w |
|---|---|
| Hydroxypropylmethyl cellulose¹⁰ | 5 |
| Terconazole | 20.0 |
| Water | 75.0 |

| | |
|---|---|
| ¹⁰ - The hydroxypropylmethyl cellulose was HPMC E50LV™ available from Dow Chemical Co. of Midland, MI. | |

### Example 19

A multi-layer composition was prepared as follows. The solution of Example 18 (before freeze-drying) was dispensed, cooled to room temperature, and carefully transferred into a 1.0 mL mold. The solution of Example 17 (before freeze-drying) was then dispensed, cooled to room temperature, and carefully transferred into the mold. The weight ratio of the solution of Example 18 to the solution of Example 17 was about 1:1. The mold and its contents were frozen rapidly in a cold gas freezing tunnel. The water in the ice state was removed by lyophilization to produce the multi-layer composition.

The dissolution rate of the multi-layer composition in a medium having a pH of about 4 was determined by the membrane dissolution method described in Example 16. The dissolution rate was determined to be about 50% by weight per 12 hours.

### Example 20

A micro-multi-layer composition was prepared as follows. The solutions of Examples 17 and 18 (before freeze-drying) were slowly mixed at a weight ratio of about 1:1 and at a temperature of 20-45° C. The mixture was carefully transferred into 1.0 mL molds. The mold and its contents were frozen rapidly in a cold gas freezing tunnel. The water in the ice state was removed by lyophilization to produce the micro-multi-layer composition.

The dissolution rate of the micro-multi-layer composition in a medium having a pH of about 4 was determined by the membrane dissolution method described in Example 16. The dissolution rate was determined to be about 50% by weight per 10 hours.

## Claims

1. A composition comprising
(a) a sustained release layer comprising
(i) a water-soluble polymer, and
(ii) a first pharmaceutically active agent; and
(b) a fast release layer comprising
(i) a matrix forming agent, and
(ii) a second pharmaceutically active agent.
said composition being obtainable by pouring a first solution and a second solution into a container, and freeze-drying the resulting solution, wherein said first solution comprises said water-soluble polymer and said first pharmaceutically active agent, and said second solution comprises said matrix forming agent and said second pharmaceutically active agent.

2. A composition as defined in claim 1, wherein the water-soluble polymer is selected from the group consisting of celluloses, cellulose ethers, polycarboxylated vinyl polymers, polyurethanes, gelatins, polysaccharide gums, seed gums, crosslinked alginate gum gels, and any combination of any of the foregoing.

3. A composition as defined in claim 2, wherein the polycarboxylated vinyl polymer is selected from the group consisting of polyacrylic acid polymers, palyacrylic acid polymers crosslinked with polyalkenyl polyethers, and any combination of any of the foregoing.

4. A composition as defined in claim 2, wherein the polysaccharide gum is selected from the group consisting of karaya gum, ghatti gum, and combination of any of the foregoing.

5. A composition as defined in claim 2, wherein the seed gum is selected from the group consisting of guar gum, locust bean gum, psyllium seed gum, and any combination of any of the foregoing.

6. A composition as defined in claim 2, wherein the water-soluble polymer is selected from the group consisting of polyurethanes, gelatins, hydroxypropylmethylcellulose, sodium carboxymethylcellulose, methylcellulose, hydroxyethylcellulose, hydroxypropylcellulose, hydroxyethylmethylcellulose, hydroxyethylethylcellulose, hydroxypropylethylcellulose, carbopol, polyvinyl alcohol, dextran, chitosan, starches, polyacrylamides, polyacrylates, agar, collagen, fibronectin, alginic acid, pectin, hyaluronic acid, and any combination of any of the foregoing.

7. A composition as defined in claim 1, wherein the sustained release layer further comprises (iii) a fatty acid.

8. A composition as defined in claim 1, wherein the sustained release layer comprises a hydrogenated vegetable oil.

9. A composition as defined in claim 1, wherein the first pharmaceutically active agent is selected from the group consisting of an anti-fungal agent, an anti-bacterial agent, a nutrient, a vitamin, a mineral, a diagnostic, a fertilizer, an insecticide, or any combination of any of the foregoing.

10. A composition as defined in claim 1, wherein the first pharmaceutically active agent is selected from the group consisting of metronidazole, miconazole nitrate, terconazole, chlorpheniramine maleate, pseudophedrine, detromethorphan, meclizine dihydrochloride, haloperidol, albuterol sulfate, dimenhydrinate, benzodiazepines, and any combination of any of the foregoing.

## Patentansprüche

1. Zusammensetzung umfassend
(a) eine verzögert freisetzende Schicht umfassend
(i) ein wasserlösliches Polymer, und
(ii) ein erstes pharmazeutisch aktives Mittel; und
(b) eine schnell freisetzende Schicht umfassend
(i) ein Matrix-bildendes Mittel, und
(ii) ein zweites pharmazeutisch aktives Mittel,
wobei die Zusammensetzung erhältlich ist durch Gießen einer ersten Lösung und einer zweiten Lösung in einen Behälter, und Gefriertrocknen der resultierenden Lösung, wobei die erste Lösung das wasserlösliche Polymer und das erste pharmazeutisch aktive Mittel umfaßt, und die zweite Lösung das Matrix-bildende Mittel und das zweite pharmazeutisch aktive Mittel umfaßt.

2. Zusammensetzung wie in Anspruch 1 definiert, wobei das wasserlösliche Polymer ausgewählt ist aus der Gruppe bestehend aus Zellulosen, Zelluloseethem, polycarboxylierten Vinylpolymeren, Polyurethanen, Gelatinen, Polysaccharidgummis, Samengummis, kreuzvemetzten Alginat-Gummigelen und jeder Kombination aus jedem der vorhergehenden.

3. Zusammensetzung wie in Anspruch 2 definiert, wobei das polycarboxylierte Vinylpolymer ausgewählt ist aus der Gruppe bestehend aus Polyacrylsäurepolymeren, Polyacrylsäurepolymeren kreuzvemetzt mit Polyalkenylpolyethem und jeder Kombination aus jedem der vorhergehenden.

4. Zusammensetzung wie in Anspruch 2 definiert, wobei der Polysaccharidgummi ausgewählt ist aus der Gruppe bestehend aus Karayagummi, Ghattigummi und der Kombination aus jedem der vorhergehenden.

5. Zusammensetzung wie in Anspruch 2 definiert, wobei der Samengummi (seed gum) ausgewählt ist aus der Gruppe bestehend aus Guarkernmehl, Johannisbrotkernmehl, Psylliumgummi und jeder Kombination aus jedem der vorhergehenden.

6. Zusammensetzung wie in Anspruch 2 definiert, wobei das wasserlösliche Polymer ausgewählt ist aus der Gruppe bestehend aus Polyurethanen, Gelatinen, Hydroxypropylmethylzellulose, Natriumcarboxymethylzellulose, Methylzellulose, Hydroxyethylzellulose, Hydroxypropylzellulose, Hydroxyethylmethylzellulose, Hydroxyethylethylzellulose, Hydroxypropylethylzellulose, Carbopol, Polyvinylalkohol, Dextran, Chitosan, Stärken, Polyacrylamiden, Polyacrylaten, Agar, Kollagen, Fibronectin, Alginsäure, Pectin, Hyaluronsäure und jede Kombination aus jedem der vorhergehenden.

7. Zusammensetzung wie in Anspruch 1 definiert, wobei die verzögert freisetzende Schicht weiterhin (iii) eine Fettsäure umfaßt.

8. Zusammensetzung wie in Anspruch 1 definiert, wobei die verzögert freisetzende Schicht ein hydrogeniertes Pflanzenöl umfaßt.

9. Zusammensetzung wie in Anspruch 1 definiert, wobei das erste pharmazeutisch aktive Mittel ausgewählt ist aus der Gruppe bestehend aus einem anti-mykotischen Mittel, einem anti-bakteriellen Mittel, einem Nährstoff, einem Vitamin, einem Mineral, einem diagnostischen Mittel, einem Düngemittel, einem Insektizid oder jeder Kombination aus jedem der vorhergehenden.

10. Zusammensetzung wie in Anspruch 1 definiert, wobei das erste pharmazeutisch aktive Mittel ausgewählt ist aus der Gruppe bestehend aus Metronidazol, Miconazolnitrat, Terconazol, Chlorpheniraminmaleat, Pseudophedrin, Detromethorphan, Meclizindihydrochlorid, Haloperidol, Albuterolsulfat, Dimenhydrinat, Benzodiazepinen und jeder Kombination aus jedem der vorhergehenden.

## Revendications

1. Composition comprenant :
(a) une couche à libération prolongée comprenant :
(i) un polymère hydrosoluble, et
(ii) un premier agent actif sur le plan pharmaceutique ; et
(b)tune couche à libération rapide comprenant :
(i) un agent formant une matrice, et
(ii) un second agent actif sur le plan pharmaceutique,
ladite composition pouvant être obtenue en versant une première solution et une seconde solution dans un récipient, et en lyophilisant la solution résultante, dans laquelle ladite première solution comprend ledit polymère hydrosoluble et ledit premier agent actif sur le plan pharmaceutique, et ladite seconde solution comprend ledit agent formant une matrice, et ledit second agent actif sur le plan pharmaceutique.

2. Composition selon la revendication 1, dans laquelle le polymère hydrosoluble est choisi dans le groupe constitué par des celluloses, des éthers de cellulose, des polymères de vinyle polycarboxylés, des polyuréthanes, des gélatines, des gommes polysaccharide, des gommes de graines, des gels de gommes alginate réticulées, et toute combinaison de tout ce qui précède.

3. Composition selon la revendication 2, dans laquelle le polymère de vinyle polycarboxylé est choisi dans le groupe constitué par des polymères du poly(acide acrylique), des polymères du poly(acide acrylique) réticulés avec des polyéthers de polyalcényle, et toute combinaison de tout ce qui précède.

4. Composition selon la revendication 2, dans laquelle la gomme polysaccharide est choisie dans le groupe constitué par la gomme karaya, la gomme ghatti, et une combinaison de tout ce qui précède.

5. Composition selon la revendication 2, dans laquelle la gomme de graines est choisie dans le groupe constitué par la gomme de guar, la gomme de caroube, la gomme de graines de psyllium, et toute combinaison de tout ce qui précède.

6. Composition selon la revendication 2, dans laquelle le polymère hydrosoluble est choisi dans le groupe constitué par des polyuréthanes, des gélatines, l'hydroxypropylméthylcellulose, la carboxyméthylcellulose de sodium, la méthylcellulose, l'hydroxyéthylcellulose, l'hydroxypropylcellulose, l'hydroxyéthylméthyl-cellulose, l'hydroxyéthyléthylcellulose, l'hydroxypropyléthylcellulose, le carbopol, l'alcool polyvinylique, le dextrane, le chitosane, des amidons, des polyacrylamides, des polyacrylates, l'agar-agar, le collagène, la fibronectine, l'acide alginique, la pectine, l'acide hyaluronique, et toute combinaison de tout ce qui précède.

7. Composition selon la revendication 1, dans laquelle la couche à libération prolongée comprend en outre (iii) un acide gras.

8. Composition selon la revendication 1, dans laquelle la couche à libération prolongée comprend une huile végétale hydrogénée.

9. Composition selon la revendication 1, dans laquelle le premier agent actif sur le plan pharmaceutique est choisi dans le groupe constitué par un agent antifongique, un agent anti-bactérien, un nutriment, une vitamine, une substance minérale, un diagnostique, un engrais, un insecticide, ou toute combinaison de tout ce qui précède.

10. Composition selon la revendication 1, dans laquelle le premier agent actif sur le plan pharmaceutique est choisi dans le groupe constitué par le métronidazole, le nitrate de miconazole, le terconazole, le maléate de chlorphéniramine, la pseudophédrine, le dextrométhorphane, le dichlorhydrate de méclizine, l'halopéridol, le sulfate d'albutérol, le dimenhydrinate, les benzodiazépines, et toute combinaison de tout ce qui précède.
